Europäisches Patentamt

European Patent Office

Office européen des brevets

(11) Numéro de publication: **0 042 321**
**B1**

# (12) FASCICULE DE BREVET EUROPEEN

(45) Date de publication du fascicule du brevet: **29.01.86**

(51) Int. Cl.⁴: **A 61 M 16/00**

(21) Numéro de dépôt: **81400888.4**

(22) Date de dépôt: **04.06.81**

(54) **Respirateur à correction automatique de ventilation.**

(30) Priorité: **10.06.80 FR 8012824**

(43) Date de publication de la demande:
**23.12.81 Bulletin 81/51**

(45) Mention de la délivrance du brevet:
**29.01.86 Bulletin 86/05**

(84) Etats contractants désignés:
**AT BE CH DE FR GB IT LI LU NL SE**

(56) Documents cités:
**FR-A-2 159 735**
**FR-A-2 178 057**
**FR-A-2 321 272**
**FR-A-2 344 278**
**US-A-3 057 346**
**US-A-3 200 816**

(73) Titulaire: **L'AIR LIQUIDE, SOCIETE ANONYME POUR L'ETUDE ET L'EXPLOITATION DES PROCEDES GEORGES CLAUDE**
**75, Quai d'Orsay**
**F-75321 Paris Cedex 07 (FR)**

(72) Inventeur: **Monnier, Jean-Pierre**
**31, avenue du Perche**
**F-78190 Maurepas (FR)**

(74) Mandataire: **Leclercq, Maurice et al**
**L'AIR LIQUIDE SOCIETE ANONYME POUR L'ETUDE ET L'EXPLOITATION DES PROCEDES GEORGES CLAUDE 75, Quai d'Orsay**
**F-75321 Paris Cedex 07 (FR)**

Courier Press, Leamington Spa, England.

EP 0 042 321 B1

# Description

La présente invention concerne un dispositif respirateur pour la ventilation artificielle des voies pulmonaires d'un utilisateur selon un cycle de phases inspiratoires et expiratoires du type comportant:

— un circuit d'alimentation délivrant en continu un gaz respirable;
— un circuit d'utilisation comportant une branche d'inspiration et une branche d'expiration munie d'une soupape d'expiration;
— un accumulateur de gaz respirable formant réserve-tampon;
— un distributeur relié audit circuit d'alimentation et comportant une soupape d'inspiration communiquant avec ladite branche d'inspiration, une vanne d'accumulation et une soupape unidirectionnelle toutes deux de communication avec l'accumulateur; et
— un dispositif de commande à action cyclique commandant, selon un programme déterminé, les dites soupapes d'inspiration, d'expiration et d'accumulation associé à un dispositif de correction de ventilation sensible aux variations de la pression dans la branche d'inspiration et agencé pour diriger vers la branche d'inspiration pendant les phases d'inspiration le gaz respirable venant du circuit d'alimentation et de l'accumulateur, pendant les phases d'expiration le gaz respirable venant du circuit d'alimentation vers l'accumulateur.

Ces respirateurs, dans lesquels du gaz respirable est stocké pendant la phase d'expiration puis restitué au circuit d'utilisation pendant la phase d'inspiration, sont destinés principalement au traitement des insuffisances respiratoires et peuvent être utilisés en milieu hospitalier, à domicile, ou encore en secourisme.

Le dispositif de commande à action cyclique permet de délivrer à l'utilisateur une ventilation, c'est-à-dire une quantité donnée de gaz respirable par unité de temps, dont les paramètres, en particulier la fréquence des cycles respiratoires, la durée des temps inspiratoires et expiratoires et le rapport entre ces temps inspiratoires et expiratoires sont affichés et modifiables à volonté.

La ventilation peut donc être facilement contrôlée et réglée par l'opérateur chargé de la conduite du respirateur, généralement un médecin.

Les FR—A—2.321.272 et 2.344.278 décrivent des appareils de ce type.

Des études approfondies effectuées dans le domaine de la ventilation artificielle ont permis de mettre en évidence un certain nombre de difficultés qui se présentent dans la pratique médicale et les insuffisances de certains appareils actuels pour les surmonter. Ces difficultés se recontrent principalement dans deux cas:

— celui dans lequel la ventilation, réglée par l'opérateur, devient insuffisante pour le patient, et

— celui dans lequel cette ventilation devient excessive pour le patient en raison par exemple d'une obstruction de ses voies pulmonaires au cours du traitement.

La présente invention a pour but de pallier ces difficultés et propose à cet effet un respirateur du type précité mais comportant:

— un circuit auxiliaire d'adduction à vanne reliant directement le circuit d'alimentation audit accumulateur;
— un circuit d'échappement à vanne de l'accumulateur vers l'atmosphère;
— ledit dispositif de correction de ventilation étant destinée à la correction de la durée des temps inspiratoire et expiratoire et agissant sur les circuits d'adduction ou d'échappement pour effectuer un apport supplémentaire ou une évacuation d'air respirable en fonction desdites variations et de ladite durée.

Le respirateur selon l'invention permet donc d'augmenter ou de diminuer la ventilation, de façon automatique, en fonction de la pression dans le circuit d'utilisation, laquelle est tributaire du patient, c'est-à-dire de ses réactions ou de son état.

L'apport supplémentaire de gaz respirable au circuit d'utilisation se fait par l'intermédiaire de l'accumulateur avec un débit dont la valeur est fonction de l'orifice calibré et du temps d'ouverture de l'électrovanne, tandis que l'évacuation d'air respirable en excès se fait à partir de l'accumulateur, de façon contrôlée grâce à l'électrovanne.

On permet ainsi de corriger à coup sûr et automatiquement un défaut ou un excès de ventilation, et cela grâce à l'action d'un correcteur sensible aux variations de pression de la branche inspiratoire, qui commande un réglage correctif du dispositif de commande cyclique et l'ouverture de la vanne du circuit auxiliaire d'adduction ou de la vanne de circuit d'échappement.

Dans le FR—A—2.321.272, on ne retrouve pas ce double système de correction, car le groupe de compensation automatique de base vise à maintenir, une contre-pression constante dans la voie respiratoire à l'expiration. Il joue certes également le rôle de fournir un appoint de gaz respirable en cas d'inspiration spontanée du patient, mais cela sans rien changer en ce qui concerne les phase inspiratoires et expiratoires qui ont été préréglées au départ. En d'autres termes, si l'inspiration naturelle intervient avant le moment prédéterminé du début d'inspiration mécanique, le dispositif de compensation automatique permet seulement de pallier une attente de la phase normale d'inspiration par envoi de bouffées de gaz respirable. Il s'agit là d'un système de ventilation dit "assisté-contrôlé", et le groupe accélérateur ne constitue en fait qu'une valve dite "à la demande" utilisée dans le système IMV ("intermittent mandatory ventilation").

Au contraire, la présente invention prévoit d'abord et essentiellement une adaptation immédiate du cycle inspiratoire en fonction de la demande respiratoire du patient. Une détection d'une dépression a pour effet immédiat d'avancer le début de la phase inspiratoire pour l'adapter sur le champ à la nouvelle situation et simultanément d'assurer un supplément de débit de gaz respirable. De même, une détection d'une surpression dans la branche d'inspiration provoque l'anticipation de la phase expiratoire et le relâchement à l'air libre d'un débit excédentaire de gaz respiratoire. Cette double adaptation immédiate du cycle respiratoire et du débit respiratoire constitue un progrès essentiel par rapport au FR—A—2.321.272, qui ne prévoit que certaines compensations sans modifications du cycle respiratoire lui-même.

D'autres caractéristiques et avantages de l'invention apparaîtront au cours de la description qui va suivre.

Dans les dessins annexés donnés uniquement à titre d'exemple:

— la figure 1 est un diagramme montrant la valeur, en fonction du temps $t$, de la pression $p$ dans le circuit d'utilisation d'un respirateur de type connu dans le cas où la ventilation délivrée par ledit respirateur devient insuffisante.
— la figure 2 est un diagramme identique à celui de la figure 1 mais relatif aux cas où la ventilation délivrée devient excessive.
— la figure 3 représente de façon schématique, un respirateur selon l'invention.
— la figure 4 représente, à plus grande échelle et de façon plus détaillée, le dispositif de correction de ventilation.
— la figure 5 est un diagramme identique à celui de la figure 1 mais obtenu avec le respirateur selon l'invention.
— la figure 6 est un diagramme identique à celui de la figure 2 mais obtenu avec le respirateur selon l'invention.

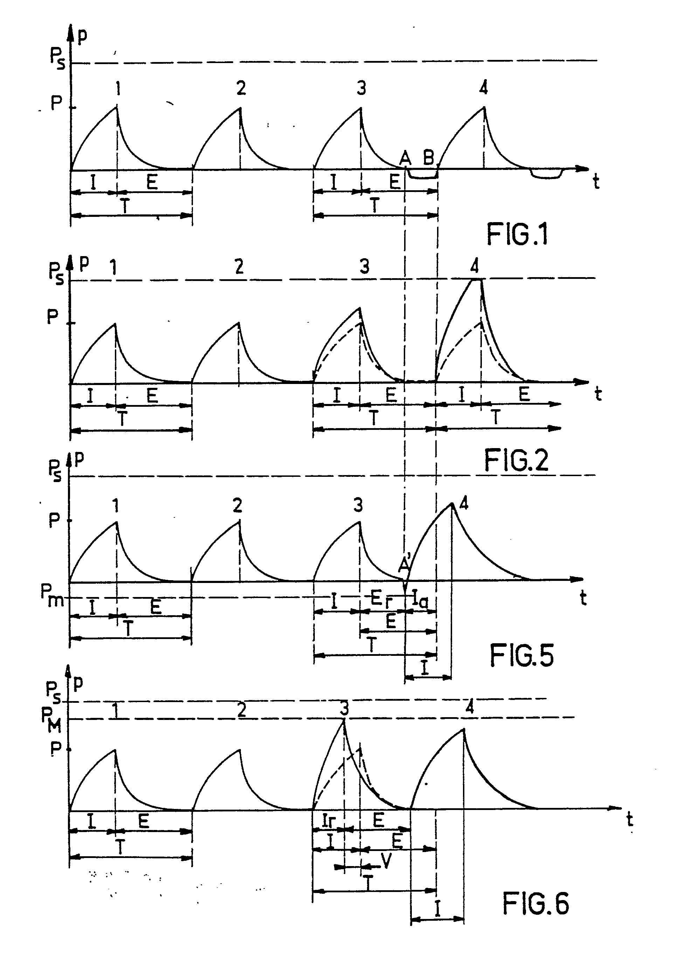

En se référant à la figure 1, on voit que la pression dans le circuit d'utilisation, ou circuit patient, croît pendant la durée I de la phase d'inspiration, ou temps inspiratoire, depuis une valeur prise pour origine (pression pulmonaire résiduelle) jusqu'à une valeur maximale P puis décroît pendant la durée E de la phase expiratoire, ou temps expiratoire, jusqu'à la valeur d'origine, la somme de ces durées étant la période respiratoire T(I+E=T). Il est à noter que la pression dans le circuit d'utilisation ne peut en aucun cas dépasser une valeur de sécurité Ps, ceci grâce à une soupape de sécurité prévue dans ledit circuit. Les valeurs I, E, T, ainsi que la quantité de gaz insufflée, sont réglées par l'opérateur de façon à ce qu'elles soient conformes aux besoins et aux caractéristiques du patient. La valeur de P résulte de ce réglage. Les cycles respiratoires correspondants sont représentés en 1 et 2.

Si la ventilation devient insuffisante par suite de modifications des caractéristiques de patient, celui-ci réagit par un effort inspiratoire pendant la phase expiratoire, c'est-à-dire entre le moment où il a fini d'expirer (A) et le début de la phase enspiratoire suivante (B), imposée par le respirateur. Cet effort inspiratoire crée dans le circuit patient, entre A et B, une dépression qui traduit l'insuffisance de ventilation (cycles 3 et 4).

La figure 2 dans laquelle les mêmes lettres désignent les mêmes paramètres que dans la figure 1, est relative au cas d'un patient dont les voies pulmonaires sont obstruées. Dans un premier temps (cycle 3), la pression s'élève fortement dans le circuit patient. Ceci se traduit par une augmentation du niveau d'énergie dans l'accumulateur et une augmentation de la pression qui se pour suit à chaque cycle jusqu'à atteindre la valeur de la pression de sécurité Ps (cycle 4). La ventilation délivrée par le respirateur est trop importante.

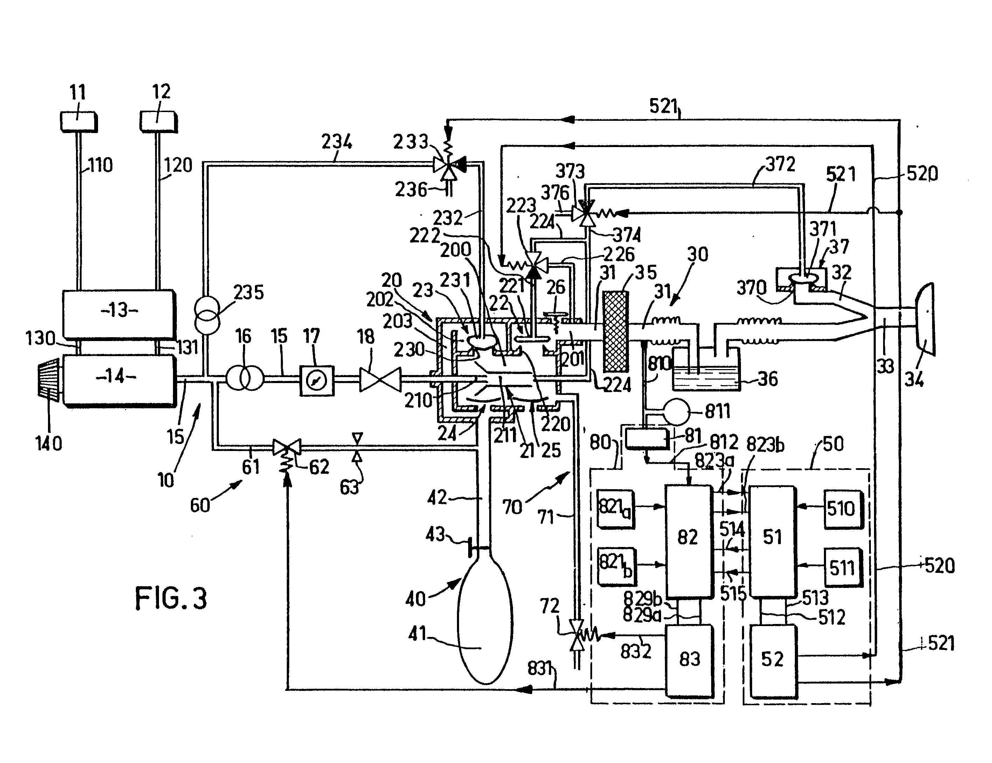

Selon le mode de réalisation représenté à la figure 3, le respirateur selon l'invention comporte essentiellement un circuit d'alimentation en gaz respirable 10, un bloc distributeur 20, un circuit d'utilisation ou circuit patient 30, un accumulateur pneumatique 40, ce circuit 30 et cet accumulateur recevant le gaz respirable du circuit 10 par l'intermédiaire du distributeur 20, et un dispositif de commande électrique à action cyclique (50), dont la fonction est de contrôler les cycles successifs d'inspiration et d'expiration et d'assurer la respiration du patient.

Le circuit d'alimentation 10 comporte deux sources 11 et 12 de gaz sous pression, par exemple, dans le cas où le patient doit être ventilé en air suroxygéné, une source d'air et une source d'oxygène.

Les sources 11 et 12 communiquent, par des conduits 110 et 120, avec un égalisateur de pression 13 qui communique lui-même, par des conduits 130 et 131, avec un mélangeur 14 muni d'un bouton de réglage 140 qui permet le dosage du mélange des deux gaz.

Le mélange 14 communique avec une canalisation 15 munie d'un détendeur 16 qui ramène la pression du gaz à une valeur stable, de l'ordre de 1 bar, d'un débitmètre 17 et d'un robinet de réglage de débit 18.

Le distributeur 20 comporte un venturi 21 dont l'injecteur 210, relié à la canalisation 15, reçoit le gaz respirable et dont le divergent 211 débouche directement dans un espace interne 200 du distributeur. Ledit espace 200 communique avec le circuit d'utilisation 30 par une vanne d'inspiration 22 logée dans un compartiment 201, avec l'accumulateur 40 d'une part par l'intermédiaire d'une vanne d'accumulation 23 logée dans un compartiment 202 communiquant avec un passage 203 et d'autre part par une soupape unidirectionnelle 24 (laissant passer le gaz dans le sens allant de l'accumulateur 40 vers le circuit 30) et enfin avec l'atmosphère avec une soupape d'air additionnel 25. Le compartiment 201 communique avec l'atmosphère par une soupape tarée 26 qui empêche la pression dans le circuit 30 de dépasser la valeur de sécurité Ps.

La vanne d'inspiration 22 comporte un siège 220, un clapet pneumatique 221 relié, par un conduit 222, à une électrovanne 223 qui a pour fonction le gonflage et le dégonflage dudit clapet et communique à cet effet, d'une part, par un conduit 224, avec la sortie du divergent 211 du venturi et d'autre part, par un conduit 226, avec le compartiment 201 du distributeur 20.

La vanne d'accumulation 23 comporte un siège 230, un clapet pneumatique 231 qui communique, par l'intermédiaire d'un conduit 232, avec une électrovanne 233 de gonflage et de dégonflage dudit clapet. L'électrovanne 233 communique d'une part avec la canalisation 15, par l'intermédiaire d'un conduit 234 muni d'un détendeur 235, et d'autre part avec l'atmosphère par une purge 236.

Le circuit d'utilisation 30 comporte une branche d'inspiration 31 qui communique directement avec le compartiment 201 du distributeur 20 et une branche d'expiration 21, ces deux branches 31 et 32 communiquant, par l'intermédiaire d'un tronc commun 33 avec un masque 34 pour le patient. La branche 31 comporte un filtre bactériologique 35 et un humidificateur 36. La branche d'expiration 32 communique avec l'atmosphère par une vanne d'expiration 37. La vanne d'expiration 37 comporte un siège 370, un clapet pneumatique 371 qui communique, par un conduit 372, avec une électrovanne 373 de gonflage et de dégonflage dudit clapet. L'électrovanne 373 est reliée d'une part à la sortie du divergent 211 par l'intermédiaire d'un conduit 374 et du conduit précité 224 de la vanne d'inspiration et d'autre part avec l'atmosphère par une purge 376.

L'accumulateur 40 est constitué d'un ballon élastique 41, dit ballon intégrateur, prévu pour stocker une partie de gaz respirable formant ainsi une réserve tampon et qui est relié au distributeur 20 par une tubulure 42 munie d'une soupape de sécurité 43.

Le dispositif de commande 50 comporte essentiellement une horloge électronique 51 et un organe de contrôle 52 constitué par exemple par un amplificateur de puissance et piloté par ladite horloge par l'intermédiaire des conducteurs 512 et 513. Le dispositif 50 est prévu pour commander, selon un programme déterminé, la fréquence des cycles respiratoires

$$\frac{1}{I+E}$$

et le rapport I/E du temps inspiratoire sur le temps expiratoire pour chaque cycle respiratoire, cette fréquence et ce rapport étant réglables au moyen d'organes de réglage 510 et 511 respectivement, constitués par exemple par des potentiomètres et associés à l'horloge 51. L'organe de contrôle 52 est relié, par un conducteur 520, à l'électrovanne 223 et par un conducteur commun 521 aux électrovannes 233 et 373 et il est prévu pour envoyer aux dites électrovannes, en fonction des signaux de pilotage qu'il reçoit de l'horloge 51, des signaux qui commandent leur ouverture et leur fermeture et par conséquent l'ouverture et la fermeture des vannes pneumatiques 22, 23 et 37.

L'opérateur peut donc imposer au patient une suite de temps inspiratoires et expiratoires déterminés.

Le respirateur selon l'invention comporte, outre les constituants sus-mentionnés, un circuit auxiliaire d'adduction 60, un circuit d'échappement 70 et un dispositif de correction de ventilation 80.

Le circuit auxiliaire d'adduction 60 comporte essentiellement un conduit 61 muni d'une électrovanne 62 qui fonctionne par tout ou rien et d'un orifice calibré 63. Le conduit 61 relie directement la canalisation 15 du circuit d'alimentation 10 à la tubulure 42 de l'accumulateur 40.

Le circuit d'échappement 70 comporte une canalisation 71 de mise à l'atmosphère reliée à l'espace interne 200 du distributeur 20 et munie, à son extrémité, d'une électrovanne 72 qui fonctionne par tout ou rien.

Le dispositif de correction de ventilation 80 comporte un capteur de pression 81, un calculateur électronique 82 sensible aux indications dudit capteur et un organe de contrôle 83, piloté par ledit calculateur.

Le capteur 81, par exemple du type piezorésistif, est relié par un conduit 810 muni d'un manomètre 811, à la branche d'inspiration 31, il détecte la pression dans ladite branche et délivre au calculateur 82, par l'intermédiaire du conducteur électrique 812, une tension électrique proportionnelle à ladite pression.

Le calculateur 82 est prévu pour commander l'horloge 51 de façon qu'elle déclenche, lorsque la pression dans la branche 31 atteint une valeur minimale Pm déterminée (dépression) ou une valeur maximale $P_M$ déterminée (inférieure ou au plus égale à la pression de sécurité Ps), une phase inspiratoire ou une phase expiratoire anticipée, c'est-à-dire en avance sur la phase inspiratoire ou expiratoire qui se serait normalement produite selon le programme déterminé par les organes de réglage 510 et 511. Les organes 510 et 511 déterminent en effet la fréquence

$$\frac{1}{I+E}$$

des cycles respiratoires ainsi que le rapport I/E du temps inspiratoires sur le temps expiratoire, ils déterminent par conséquent I et E. Le fait de déclencher par anticipation une phase inspiratoire ou une phase expiratoire a pour conséquence de raccourcir le temps expiratoire ou le temps inspiratoire du cycle respiratoire programmé, ces deux temps prenant alors des valeurs Er et Ir, inférieures a E et I respectivement.

Le calculateur 82 est prévu également pour mettre en memoire les temps I et E et pour déterminer les différences E-Er et I-Ir de façon à commander la durée d'ouverture des vannes 62 et 72 en fonction de ces différences de temps E-Er et I-Ir.

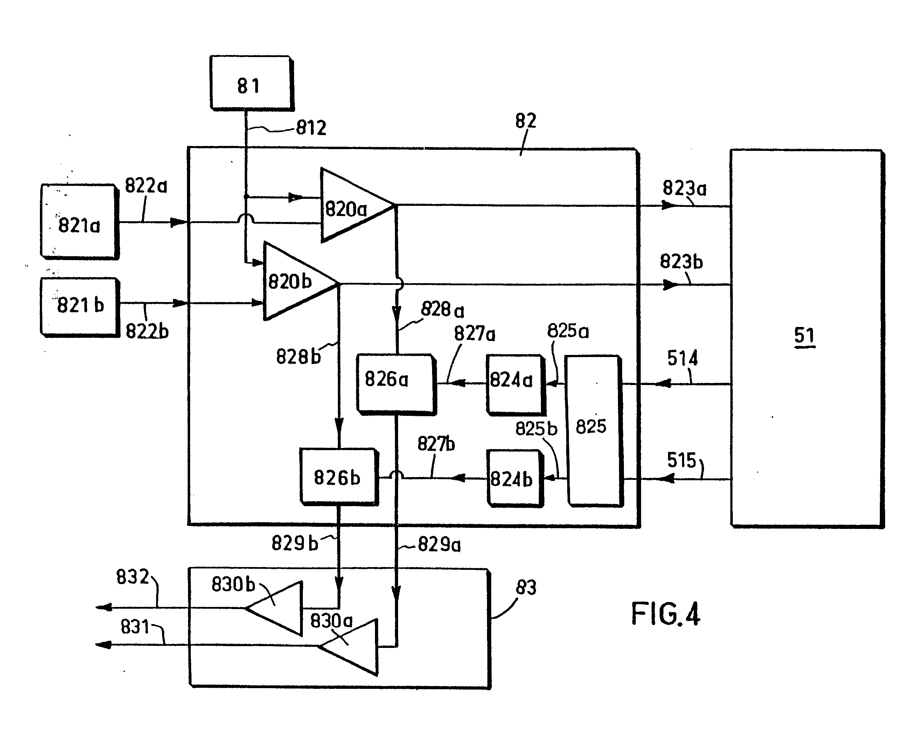

Le calculateur 82 comporte (figure 4) deux circuits comparateurs 820 a et 820 b pour comparer les tensions électriques délivrées par le capteur 81 à deux tensions de référence, l'une minimale, correspondant à la pression minimale Pm, l'autre maximale, correspondant à la pression maximale $P_M$. Ces deux tensions de référence, et par conséquent les deux pressions Pm et $P_M$, peuvent être choisies à volonté par l'opérateur (avec la condition $P_M < P_S$) au moyen des organes de réglage 821 a et 821 b, constitués par exemple par des potentiomètres, et reliés, par des conducteurs 822 a et 822 b, à l'une des entrées de chacun des comparateurs. Les comparateurs 820 a et 820 b ont leur autre entrée reliée à la sortie du capteur 81 par un conducteur 812 et leur sortie reliée, par des conducteurs 823 a et 823 b, à la base de temps (non représentée) de l'horloge 51.

Le calculateur 82 comporte également deux circuits de mémoire 824 a et 824 b et deux circuits soustracteurs 826 a et 826 b. Les circuits 824 a et 824 b reçoivent, à leur entrée, les temps I et E programmés par l'horloge 51 et les mettent en mémoire.

Ces temps I et E sont calculés par un organe de calcul 825, relié par des conducteurs 514 et 515, aux circuits (non représentés) de l'horloge 51 qui délivrent les valeurs

$$\frac{1}{I+E} \quad \text{et} \quad \frac{I}{E}$$

et sont adressés aux mémoires 824 a et 824 b par les conducteurs 825 a et 825 b. Les soustracteurs 826 a et 826 b reçoivent d'une part les temps I et E des mémoires 824 a et 824 b par l'intermédiaire des conducteurs 827 a et 827 b et d'autre part les temps Ir et Er des comparateurs 820 a et 820 b par l'intermédiaire des conducteurs 828 a et 828 b. Les différences I—Ir et E—Er sont adressées, par des conducteurs 829 a et 829 b, à l'organe de contrôle 83.

L'organe de contrôle 83 comporte deux amplificateurs 830 a et 830 b dont les entrée sont reliées aux soustracteurs 826 a et 826 b par les conducteurs précités 829 a et 829 b, leurs sorties étant reliées aux électrovannes 62 et 72 par l'intermédiaire des conducteurs 831 et 832 respectivement, pour commander l'ouverture et la fermeture des dites électrovannes en fonction de signaux qu'ils reçoivent des soustracteurs 826 a et 826 b.

Le fonctionnement du distributeur selon l'invention est le suivant:

Les vannes 22, 23 et 37, commandées par le dispositif de commande 50, provoquent selon qu'elles sont ouvertes ou fermées, un cycle d'inspiration ou au contraire un cycle d'expiration. Pendant l'inspiration (cas représenté à la figure 3) la vanne 22 est ouverte et les vannes 23 et 37 fermées. Le gaz respirable venant du circuit d'alimentation 10 par le venturi 21 et celui venant du ballon 41 par l'intermédiaire de la soupape unidirectionnelle 24 passent de l'espace interne 200 dans le compartiment 201 du distributeur 20, puis dans la branche 31 du circuit d'utilisation 30. Pendant l'expiration, la vanne 22 est fermée et les vannes 23 et 37 ouvertes. Le gaz expiré par le patient passe dans l'atmosphère par la branche d'expiration 32 et la vanne 37 tandis que le gaz respirable venant du circuit d'alimentation 10 par le venturi 21 passe de l'espace interne 200 au ballon 41 par l'intermédiaire du compartiment 202, du passage 203 et de la tubulure 42. Si la ventilation ainsi délivrée au patient est compatible en fréquence et en quantité avec les besoins et les caractéristiques pulmonaires du patient, c'est-à-dire si cette ventilation se fait selon les cycles 1 et 2 des figures 5 et 6, les électrovannes 62 et 72 associées au circuit d'adduction 60 et au circuit d'échappement 70 sont maintenues fermées par le dispositif de correction 80.

Si la ventilation devenant insuffisante, le patient fait un effort inspiratoire, il s'ensuit dans la branche 31 du circuit d'utilisation une dépression qui apparaît en A' sur la figure 5 (correspondant au point A de la figure 1). Cette dépression, détectée par le capteur 81, est convertie en une tension électrique proportionnelle qui est reçue par les comparateurs 820 a et 820 b. Cette tension est comparée aux tensions de référence réglées par les organes de réglage 821 a et 821 b et dès qu'elle atteint la valeur de la tension de référence réglée par 821 a et correspondant à Pm, le comparateur 820 a envoie un signal électrique à l'horloge 51 par le conducteur 823 a et au soustracteur 826 a, par le conducteur 827 a. L'horloge 51 pilote l'organe de contrôle 52 pour qu'il déclenche une phase inspiratoire anticipée. Le temps expiratoire est donc raccourci à une valeur réelle Er inférieure au temps E programmé par le dispositif 50. Le soustracteur 826 a détermine la différence entre le temps expiratoire E mis en mémoire qui aurait dû avoir lieu et le temps expiratoire Er qui s'est produit. La différence des deux temps permet au soustracteur 826 a de commander, par l'intermédiaire de l'amplificateur 830 a, l'ouverture de l'électrovanne 62 pendant une durée E−Er=Ia et d'alimenter le ballon 41 en gaz respirable d'appoint. Le niveau d'énergie dans le ballon intégrateur 41 augmente, ce qui entraîne une augmentation du débit d'inspiration. La ventilation se fait alors selon les cycles 3 et 4 de la figure 5. Le cycle 4 empiète sur le cycle 3 et sa pression de pointe dépasse la valeur P des cycles précédents.

Si, au contraire, la ventilation devient excessive par suite d'une obstruction des voies pulmonaires du patient, il s'ensuit une augmentation de pression dans le circuit d'utilisation. Cette pression détectée par le capteur 81 est convertie en tension électrique proportionnelle qui est reçue par les comparateurs 820 a, 820 b. Dès que cette tension atteint la valeur de référence réglée par 821 b et correspondant à $P_M$, le comparateur 820 b envoie un signal électrique à l'horloge 51 par le conducteur 823 b et au soustracteur 826 b par le conducteur 827 b. L'horloge 51 pilote l'organe de contrôle 52 pour qu'il déclenche une phase

expiratoîre anticipée. Le temps inspiratoire est donc raccourci à une valeur réelle Ir inférieure au temps inspiratoire I programmé par le dispositif 50. Le soustracteur 826 b détermine également la différence entre le temps inspiratoire I mis en mémoire qui aurait dû avoir lieu et le temps inspiratoire Ir qui s'est produit. La différence des deux temps permet au soustracteur 826 b, par l'intermédiaire de l'amplificateur 830 b, de commander l'ouverture de l'électrovanne 72 pendant une durée I−Ir=V, de façon à rejeter à l'extérieur une partie du gaz respirable stocké dans le ballon 41, provoquant sa décompression partielle. Le niveau d'énergie dans le ballon 41 diminue et par conséquent le débit d'inspiration diminue également.

Les cycles d'inspiration et d'expiration présentent alors la forme représentée par les cycles 3 et 4 de la figure 6. La pression de pointe du cycle 3 atteint la valeur $P_M$, tandis que la pression de pointe du cycle 4 est ramenée à une valeur inférieure à $P_M$, en raison de la décompression du ballon 41.

On pourrait alors apporter au mode de réalisation décrit et représenté de nombreuses variantes sans pour autant sortir du cadre de l'invention.

Le damande divisionaire No. 84—20—0213 (EP 0 127 905) comprend, telle que déposée, une description similaire à la description de la présente demande et des revendications concernant un circuit d'échappement (70) et un dispositif de correction de ventilation (80) sensible aux variations de la pression.

**Revendications**

1. Respirateur pour la ventilation artificielle des voies pulmonaires d'un utilisateurs selon un cycle de phases inspiratoires et expiratoires du type comportant un circuit d'alimentation (10) délivrant en continu un gaz respirable; un circuit d'utilisation (30) comportant une branche d'inspiration (31) et une branche d'expiration (32) munie d'une soupape d'expiration (37); un accumulateur de gaz respirable (40) formant réserve-tampon; un distributeur (20) relié audit circuit d'alimentation (10) et comportant une soupape d'inspiration (22) communiquant avec ladite branche d'inspiration (31), une vanne d'accumulation (23) et une soupape unidirectionnelle (24) toutes deux de communication avec l'accumulateur (40) et un dispositif de commande à action cyclique (50) commandant, selon un programme déterminé, lesdites soupapes d'inspiration (22), d'expiration (37) et d'accumulation (23) associé à un dispositif de correction de ventilation (80) sensible aux variations de la pression dans la branche d'inspiration (31) et agencé pour diriger vers la branche d'inspiration (31) pendant les phase d'inspiration le gaz respirable venant du circuit d'alimentation (10) et de l'accumulateur (40), pendant les phases d'expiration le gaz respirable venant du circuit d'alimentation (10) vers l'accumulateur (40), caractérisé par:

— un circuit auxiliaire d'adduction (60) à vanne (62) reliant directement le circuit d'alimentation (10) audit accumulateur (40);
— un circuit d'échappement (70) à vanne (72) de l'accumulateur (40) vers l'atmosphère;
— ledit dispositif de correction de ventilation (80) étant destiné à la correction de la durée des temps inspiratoire et expiratoire et agissant sur les circuits d'adduction (60) ou d'échappement (70) pour effectuer un apport supplémentaire ou une évacuation d'air respirable en fonction des dites variations et de ladite durée.

2. Respirateur selon la revendication 1, caractérisé en ce que le conduit (61) et la canalisation (71) précités communiquent avec ledit accumulateur (40) respectivement en amont et en aval de la soupape unidirectionnelle (24) pour le sens d'écoulement du gaz dudit accumulateur (40) vers ladite branche d'inspiration (31).

3. Respirateur selon la revendication 2, caractérisé en ce que le dispositif de correction de ventilation (80) précité comporte un capteur de pression (81) prévu pour délivrer des signaux électriques en fonction de la pression dans la branche d'inspiration (31), un calculateur électronique (82) relié audit capteur ainsi qu'au dispositif de commande à action cyclique (50) et un organe de contrôle (83) piloté par ledit calculateur et prévu pour commander l'ouverture et la fermeture des électrovannes (62, 72) précitées.

4. Respirateur selon la revendication 3, caractérisé en ce que le capteur de pression (81) précité est du type piezo-résistif et prévu pour délivrer une tension proportionnelle à la pression qu'il reçoit de la branche d'inspiration (31).

5. Respirateur selon la revendication 4, caractérisé en ce que le calculateur électronique (82) comporte des circuits comparateurs (820 a, 820 b) ayant l'une de leurs entrées reliée au capteur (81) et leur autre entrée reliée à des organes de réglage (821 a, 821 b) prévus pour délivrer deux tensions de référence, l'une minimale, l'autre maximale, lesdits comparateurs pilotant l'organe de commande à action cyclique (50) pour qu'il déclenche une phase inspiratoire ou une phase expiratoire lorsque la tension électrique délivrée par ledit capteur (81) atteint la valeur minimale ou la valeur maximale précitée.

6. Respirateur selon la revendication 5, caractérisé en ce que le calculateur (82) comporte des circuits de mémoire (824 a, 824 b) pour la mise en mémoire des temps inspiratoire I et expiratoire E programmés par l'organe de commande (50) et des circuits soustracteurs (826 a, 826 b) ayant l'une de leurs entrées reliée aux dites mémoires et leur autre entrée reliée aux comparateurs (820 a, 820 b) qui délivrent des temps inspiratoire Ir et expiratoire Er inférieurs à I et E et résultant du déclenchement de l'organe de commande (50) en fonction des valeurs de référence minimale et maximale précitées, les dits soustracteurs (826 a, 826 b) pilotant l'organe de contrôle (83) pour qu'il commande la durée d'ouverture des électrovannes (62, 72) en fonction des différences I-Ir et E-Er.

7. Respirateur selon la revendication 6, caractérisé en ce que l'organe de contrôle (83) comporte des circuits amplificateurs (830 a, 830 b) ayant leurs entrées reliées aux soustracteurs précités (826 a, 826 b) et leurs sorties reliées aux électrovannes (62, 72) précitées.

## Patentansprüche

1. Beatmungsgerät für die künstliche Belüftung von Lungenwegen eines Benutzers gemäß einem Zyklus mit Einatmungs- und Ausmatmungsphasen, mit einem Versorgungskreislauf (10), welcher fortlaufend ein atembares Gas zuführt; einem Benutzungskreislauf (30) mit einem Einatmungszweig (31) und einem Ausatmungszweig (32), der mit einem Ausatmungsventil (37) versehen ist; einem Speicher (40) für atembares Gas, welcher ein Reservepuffer bildet; einem Verteiler (20), der mit dem Versorgungskreislauf (10) verbunden ist und ein Einatmungsventil (22) aufweist, welches mit dem Einatmungszweig (31) in Verbindung steht, einem Speicherventil (23) und einem in nur einer Richtung wirkenden Ventil (24), welche beide mit dem Speicher (40) in Verbindung stehen, sowie einer Steuervorrichtung (50) mit zyklischem Betrieb zum Steuern der Einatmungs- (22), Ausatmungsventile (37) und eines Speichers (23) nach einem bestimmten Programm, welche einer Belüftungskorrekturvorrichtung (80) zugeordnet ist, die auf Druckveränderungen im Einatmungszweig (31) anspricht, und die eingerichtet ist, um während der Einatmungsphasen das aus dem Versorgungskreislauf (10) und dem Speicher (40) kommende, atembare Gas zum Einatmungszweig (31) zu führen und während der Ausatmungsphasen das aus dem Versorgungskreislauf (10) kommende, atembare Gas zum Speicher (40) zu führen, gekennzeichnet durch:

— einen Hilfszuführkreislauf (60) mit einem Ventil (62), welches den Versorgungskreislauf (10) direkt mit dem Speicher (40) verbindet;
— einen Ausströmungskreislauf (70) mit Ventil (72) vom Speicher (40) zur Atmosphäre hin;
— wobei die Belüftungskorrekturvorrichtung (80) bestimmt ist für die Korrektur der Dauer der Einatmungs- und Ausatmungszeiten und auf die Zuführ- (60) oder Ausströmungskreisläufe (70) derart wirkt, daß eine zusätzliche Anlieferung oder ein Abziehen atembarer Luft in Funktion der Veränderungen der genannten Dauer erfolgt.

2. Beatmungsgerät nach Anspruch 1, dadurch gekennzeichnet, daß die Leitung (61) und das Leitungssystem (71) mit dem Speicher (40) aufstromig bzw. abstromig des nur in einer Richtung wirkenden Ventils (24) für die Ausströmrichtung des Gases des Speichers (40) zu dem Einatmungszweig (31) hin in Verbindung stehen.

3. Beatmungsgerät nach Anspruch 2, dadurch gekennzeichnet, daß die Belüftungskorrekturvorrichtung (80) einen Druckmeßfühler (81) aufweist, der vorgesehen ist für das Liefern von elektrischen Signalen in Funktion des Druckes im Einatmungszweig (31), einen elektronischen Rechner (82) aufweist, der mit dem Meßfühler sowie mit der Steuervorrichtung mit zyklischem Betrieb (50) verbunden ist, und ein Kontroll- bzw. Steuerorgan (83) aufweist, welches durch den Rechner gesteuert wird und für das Steuern des Öffnens und des Schließens der genannten Elektroventile (62, 72) vorgesehen ist.

4. Beatmungsgerät nach Anspruch 3, dadurch gekennzeichnet, daß der Druckmeßfühler (81) vom Piezowiderstandstyp ist und vorgesehen ist, um eine Spannung zu liefern, die dem Druck proportional ist, welcher vom Einatmungszweig (31) aufgenommen wird.

5. Beatmungsgerät nach Anspruch 4, dadurch gekennzeichnet, daß der elektronische Rechner (82) Vergleichsschaltungen (820 a, 820 b) aufweist, deren einer Eingang mit dem Meßfühler (81) verbunden ist, und deren anderer Eingang mit Regelorganen (821 a, 821 b) verbunden ist, die vorgesehen sind, um zwei Bezugsspannungen zu liefern, wobei die eine minimal und die andere maximal ist, wobei die Vergleichsschaltungen das Steuerorgan (50) mit zyklischem Betrieb steuern, damit es eine Einatmungsphase oder eine Ausatmungsphase auslöst, wenn die von dem Meßfühler (81) gelieferte elektrische Spannung den minimalen oder den maximalen Wert annimmt.

6. Beatmungsgerät nach Anspruch 5, dadurch gekennzeichnet, daß der Rechner (82) Speicherschaltkreise (824 a, 824 b) aufweist zum Speichern der Einatmungs- I und Ausatmungszeiten E, welche durch das Steuerorgan (50) programmiert sind, sowie Subtraktionsschaltungen (826 a, 826 b), deren einer Eingang mit den Speichern und deren anderer Eingang mit den Vergleichsschaltungen (820 a, 820 b) verbunden sind, die Einatmungs- Ir und Ausatmungszeiten Er liefern, die kleiner I und E sind, und die zum Auslösen des Steuerorganes (50) in Funktion der genannten minimalen und maximalen Bezugswerte führen, wobei die Subtraktionsschaltungen (826 a, 826 b) das Steuerorgan (83) steuern, damit es die Öffnungsdauer der Elektroventile (62, 72) in Funktion der Differenzen I—Ir und E—Er steuert.

7. Beatmungsgerät nach Anspruch 6, dadurch gekennzeichnet, daß das Steuerorgan (83) Verstärkungsschaltkreise (830 a, 830 b) aufweist, deren Eingänge mit dem genannten Subtraktionsschaltungen (826 a, 826 b) und deren Ausgänge mit den genannten Elektroventilen (62, 72) verbunden sind.

## Claims

1. Respirator for the artificial ventilation of the lung ways of a user according to a cycle of phases of inhalation and exhalation, of the type comprising a circuit of alimentation (10) delivering continuously a breathable gas; a utilization circuit (30) comprising an inhalation branch (31) and an exhalation branch (32) provided with an exhalation valve (37); an accumulator for breathable gas (40) forming a reserve buffer; a distributor (20) connected to the circuit of alimentation (10) and comprising an inhalation valve (22) communicating with the said inhalation branch (31), a valve of accumulation (23) and a unidirectional valve (24) both in communication with the accumulator (40) and a commanding device with cyclic action (50) commanding, according to the predetermined programm, the said valves of inhalation (22), exhalation (37) and accumulation (23), associated to a correcting device for the ventilation (80) sensible to variations of the pressure in the inhalation branch (31) and arranged to direct the breathable gas coming from the circuit of alimentation (10) and the accumulator (40) towards the inhalation branch (31) during the inhalation phases and directing the breathable gas coming from the circuit of alimentation (10) towards the accumulator (40) during the exhalation phases, caracterized by:

— an auxiliary supply circuit (60) with a valve (62) connecting directly the circuit of alimentation (10) to the accumulator (40),
— a circuit of escapement (70) with a valve (72) from the accumulator (40) towards the atmosphere;
— the correcting device for the ventilation (80) being defined to correct the duration of inhalation and exhalation time and acting on the circuits of supply (60) or escapement (70) to effect a supplementary supply or a discharge of breathable air in response to the said variations and the said duration.

2. Respirator according to claim 1, characterized in that the conduit (61) and the canalisation (71) communicate with the said accumulator (40) upstream and downstream respectively of the unidirectional valve (24) for the outlet direction of the gas of the said accumulator (40) towards the said inhalation branch (31).

3. Respirator according to claim 2, characterized by that the device for correcting the ventilation (80) comprises a pressure sensor (81) provided to deliver electrical signals in response to the pressure in the inhalation branch (31), an electronical calculator (82) connected to the sensor as well as to the commanding device with cyclic action (50) and a control member (83) driven by the said calculator and provided for commanding the opening and the closing of the afore mentioned solenoid valves (62, 72).

4. Respirator according to claim 3, characterized by that the pressure sensor (81) is of the piezo-resistive type and is provided to deliver a voltage proportional to the pressure received from the inhalation branch (31).

5. Respirator according to claim 4, characterized by that the electronical calculator (82) comprises comparator circuits (820 a, 820 b) having the one of their inlets connected to the sensor (81) and having their other inlet connected to regulating members (821 a, 821 b) provided to deliver two reference voltages, the one minimum, the other maximum, the said comparators driving the commanding member with cyclic action (50) to release an inhalation phase or an exhalation phase during the electrical voltage delivered by the said sensor (81) reaching the afore mentioned minimum value or the maximum value.

6. Respirator according to claim 5, characterized by that the calculator (82) comprises memory circuits (824 a, 824 b) for storing of the time periods of inhalation I and exhalation E, programmed by the commanding member (50) and the subtraction circuits (826 a, 826 b) having the one of their inlets connected to the said memories and having the other inlet connected to the comparators (820 a, 820 b) delivering times of inhalation Ir and exhalation Er smaller than I and E and resulting a release of the commanding member (50) responding to the said minimum and maximum reference values, the said subtractors (826 a, 826 b) driving the control member (83) in order to command the duration of opening of the solenoid valves (62, 72) responding to the differences I—Ir and E—Er.

7. Respirator according to claim 6, characterized by that the control member (83) comprises amplification circuits (830 a, 830 b) having their inlets connected to the said subtractors (826 a, 826 b) and their exits connected to the afore mentioned solenoid valves· (62, 72).

FIG.1

FIG.2

FIG.5

FIG.6

FIG.3

0 042 321

FIG.4

0 042 321